# EUROPEAN PATENT APPLICATION

(11) **EP 1 738 724 A1**
(43) Date of publication of application: **03.01.2007**
(21) Application number: 05380144.5
(22) Date of filing: 30.06.2005
(51) Int. Cl.: A61F 5/00

(54) **Cervical collar**

(71) Applicant: Barcelona Orthopedic Necklace S.L., 08290 Cerdanyola del Vallès Barcelona (ES)
(72) Inventor: Barril Porcar, Miguel Angel, 08100 Mollet del Vallès Barcelona (ES)
(74) Representative: Curell Aguilà, Marcelino

(57) **Abstract**

Portable cervical collar suitable for exerting a therapeutical cervical traction comprising an upper flexible body, a lower flexible body and extension mechanisms (5, 6a, 6b) comprising an upper rigid part (7, 8) which rests in the upper flexible body, a lower rigid part (9, 10) which rests in the lower flexible body (3) and a manually adjustable actuator for adjusting and maintaining the relative vertical position between upper (7, 8) and lower (9, 10) rigid parts. The collar comprises three of said extension mechanisms (5, 6a, 6b): an anterior extension mechanism (5) suitable for applying an extension between the chin and the anterior pectoral area of a user and two rear extension mechanisms (6a, 6b) suitable for applying an extension between the occipital area of the head and the rear scapular area of the trunk of said user.

## Description

### Field of the invention

This invention relates to a portable cervical collar suitable for exerting a therapeutical cervical traction, of the type comprising an upper flexible body having an upper edge foreseen to rest on the head of a user wearing the collar, a lower flexible body having a lower edge foreseen to rest in the trunk of said user, said upper and lower flexible bodies being joined together allowing a relative vertical movement, and extension mechanisms which are a part of the cervical collar and each one comprising an upper rigid part that rests in said upper flexible body, a lower rigid part that rests in said lower flexible body and a manually adjustable actuator to adjust and maintain the relative vertical position between said upper and lower rigid parts.

### State of the art

There are numerous types of orthopedic collars, of a more or less complicated configuration, suitable for immobilizing and supporting the cervical column of a patient after having suffered an injury. The main part of these collars is height adjustable, so that they can adapt to the patient's physiognomy, but they do not allow exerting a significant and controlled traction force on the cervical column.

Some collars of the type above mentioned, that is, provided with extension mechanisms allowing to adjust the height of the collar by applying a significant force in order to carry out a therapeutical action of traction of the cervical column, are known. Among them, it must be pointed out the collar disclosed by US patent application US2004/0204666, which comprises two extension mechanisms arranged in centered lateral positions of the collar. These extension mechanisms allow applying and maintaining a traction of the cervical column with an force that can be adjusted by the user or by a physiotherapist. This traction can be laterally balanced, by applying the same extension to the two extension mechanisms, or it can be laterally unbalanced, by applying a higher extension to one of the extension mechanisms. Although this possibility of a laterally unbalanced traction is interesting for the therapeutical treatment of some specific types of injuries, it is not enough in the main part of cases in which the therapist needs to have more degrees of freedom in order to adjust the type of suitable traction.

Likewise, the collars disclosed by US patents US5005563, US3364926 and US2801630 can be cited. The collar disclosed by document US5005563 only has one extension mechanism in its rear portion, and thus it offers the possibility of applying an unbalanced therapeutical traction. In the collar disclosed by document US3364926, which has the particular feature that it allows the patient to freely perform a rotation movement of the head, the force applied by the extension mechanism is made by springs, so that said force is not adjustable. Finally, the collar disclosed by document US2801630 has two extension mechanisms arranged in the lateral-front area of the collar and the extension of which is not adjustable when the collar is in use position. In any case, this collar neither allows applying a therapeutical traction that is unbalanced and adjustable with accuracy.

### Summary of the invention

The objective of the present invention is to provide a portable cervical collar suitable for applying a therapeutical cervical traction and allowing a physiotherapist or a physician, without using means external to the collar, to adjust the cervical traction with more accuracy and with more degrees of freedom than in the known collars.

This objective is achieved by means of a cervical collar of the type above mentioned, characterized in that it comprises three of said extension mechanisms: one anterior extension mechanism arranged in an anterior position centered with respect to said longitudinal medium plane of said collar, and two rear extension mechanisms arranged in rear positions decentered and symetric with respec to said longitudinal medium plane of the collar, so that said anterior extension mechanism is suitable for applying an extension between the chin and the anterior pectoral area of a user wearing the collar and said rear extension mechanisms are suitable for applying an extension between the occipital area of the head and the rear scapular area of the trunk of said user.

The collar according to the invention, thanks to the specific arrangement of its three extension mechanisms, allows the medical personnel to adjust with accuracy the cervical traction suitable for this case. Due to the fact that the collar is portable and is able to maintain during long periods of time the initially adjusted traction, the patient has a high autonomy.

It is to be noted that the collar according to the invention has a great deal of possibilities of adjustment of the exerted cervical traction. It is possible to define a perfectly balanced symmetric traction, regulating in an identical fashion the rear extension mechanisms. Then, an extension or a flexion of the chin can be obtained by adjusting the anterior extension mechanism. Furthermore, a left or right lateral inclination can be obtained by individually adjusting the rear extension mechanisms. Thus, the collar according to the invention allows applying and maintaining different types of traction according to the needs of the patient; basically, from lower to higher degree of complexity : balanced traction, traction with flexion or extension of the chin, traction with left or right lateral inclination and traction with flexion or extension of the chin and with left or right lateral inclination.

Preferably, said upper rigid part of the anterior extension mechanism has the shape of an arm which bifurcates into two symmetric branches which rest in said upper flexible body extending backwards along its upper edge. This configuration ensures a suitable lateral balance of the head in the anterior area.

Preferably, said upper flexible body defines, in an anterior position centered with respect to said longitudinal medium plane of the collar, a concave base surface suitable for supporting the chin of a user wearing the collar, said concave base surface being arranged underneath the upper edge of said upper flexible body and said upper rigid part of the anterior extension mechanism having a portion at which said part rests in said upper flexible body under said concave base surface. Thanks to this configuration, it is achieved to act both in the lateral area of the jaw and in the chin, confining the chin to a position that is comfortable for the patient and that at the same time ensures a suitable immobilization of the lower jaw with respect to the upper flexible body of the collar.

Preferably, said symmetric branches of the upper rigid part of the anterior extension mechanism have the shape of a fork defining, in the bifurcation of said symmetric branches, the area at which the upper rigid part of the anterior extension mechanism rests under said concave base surface. This geometry allows acting with the same arm on the chin and on the lateral areas of the jaw guaranteeing the lateral balance of the applied extension force.

Preferably, said lower flexible body defines, separated from its lower edge and in an anterior position centered with respect to said longitudinal medium plane of the collar, a base surface suitable for resting in the pectoral area of a user wearing the collar, and said lower rigid part of the anterior extension mechanism has a portion at which said rigid part rests in said lower flexible body at the level of said base surface. This configuration offers a particularly efficient and comfortable support on the pectoral area of the patient.

Preferably, said lower rigid part of the anterior extension mechanism has the shape of an arm which in its lower end has the shape of a loop defining said portion at which the lower rigid part rests in the lower flexible body at the level of the base surface. The arm so constituted is particularly rigid and light, and ensures a suitable distribution of the force applied on the base surface.

Preferably, the upper rigid parts of the rear extension mechanisms have the shape of an arm which bifurcates into two branches which rest in said upper flexible body extending in opposite directions along their upper edge. Likewise, preferably, the lower rigid parts of the rear extension mechanisms have the shape of an arm which bifurcates into two branches which rest in said lower flexible body extending in opposite directions along their lower edge. In this manner, a suitable distribution of the applied force is achieved, preventing stress points that could be a discomfort for the patient. On the other hand, the branches extending towards the anterior area occupy a portion of the lateral areas of the collar, so that they allow to effectively perform the left or right lateralization, while the branches extending towards the rear area practically occupy all the occipital area, so that they allow to effectively apply a pure traction on the cervical column.

Preferably, each of said lower and upper flexible bodies is molded as a single part overmolding the end portions of said lower and upper rigid parts of the extension mechanisms, obtaining thereby a compact and strong collar.

Preferably, the upper edge of said upper flexible body has the shape of a closed rim housing said branches of the upper rigid parts of the rear and anterior extension mechanisms. Furthermore, the lower edge of said lower flexible body the shape of a closed rim housing said branches of the lower rigid parts of the rear extension mechanisms. In this way, the branches of the rigid parts act very directly on the head and trunk of the patient, but as they are wrapped by said rims of the flexible bodies, said branches act without disturbing the patient.

Preferably, each of said lower and upper flexible bodies has the general shape of a flexible strap the ends of which are gathered in the rear portion of the collar and said strap having closing means of said ends themselves. The collar so constituted is readily opened by its rear portion separating the ends of the flexible straps and it is closed by attaching again said ends and fixing them by means of said closing means.

Preferably, each of said actuators comprises a manually movable slide the movement of which is mechanically linked to the relative movement of said lower and upper rigid parts, said slide being suitable for firmly keeping a relative position between said lower and upper rigid parts against a force higher than or equal to 20 N, that is an effective force for a therapeutic treatment, and said slide having a locking position of said relative movement allowing to keep said force after being adjusted.

Preferably, said lower and upper flexible bodies define two wide lateral windows, allowing access to the lateral neck areas of a user wearing the collar and a wide rear window allowing access to the cervical area of the neck of said user. These windows have the double function of making easier the sweating of the user and of allowing the medical staff to directly operate on the neck if necessary.

Preferably, each of said extension mechanisms is provided with an indicator of the force applied between the upper rigid part and the lower rigid part. This force indicator is a dynamometer that allows the medical staff to control the extension force applied in each of the extension mechanisms.

### Brief description of the drawings

Other advantages and characteristics of the invention will become evident from the following description in which, entirely non-limitatively, is described a preferential embodiment of the invention, with reference to the appended drawings. The figures show:
Fig. 1 and 2, respectively, front and end views of a cervical collar according to the invention;
Fig. 3, a lateral perspective view of said collar;
Fig. 4, a perspective view wherein the lower and upper flexible bodies are shown transparent and in dash line;
Fig 5, a lateral view of said collar;
Fig. 6, a partial sectional view of the anterior portion of said collar.

### Detailed description of an embodiment of the invention

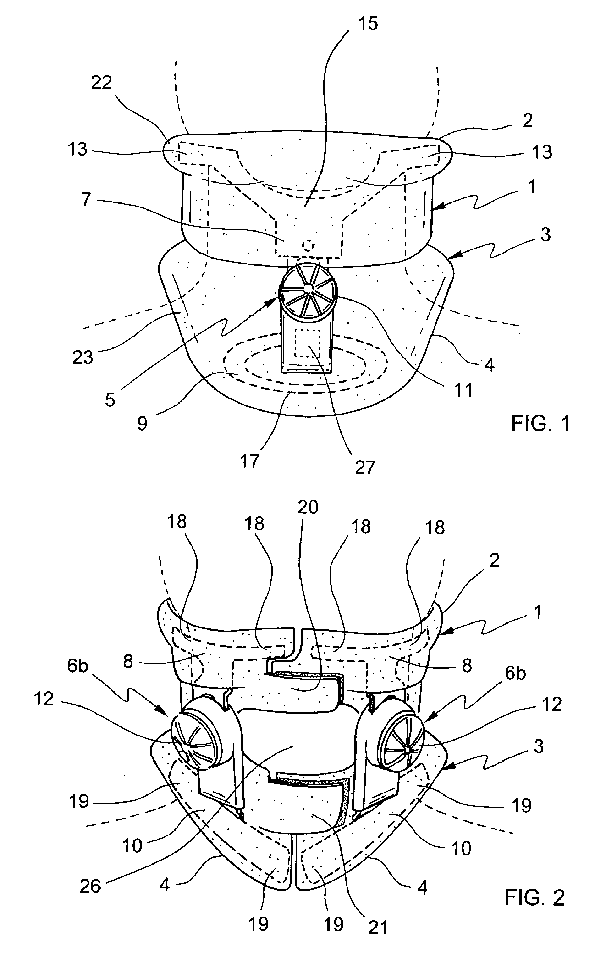

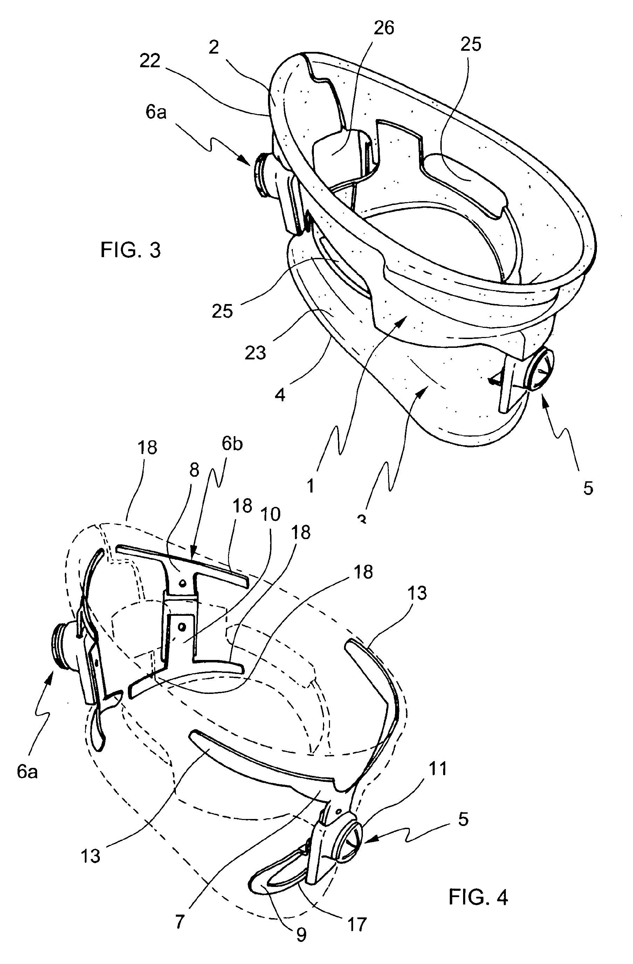

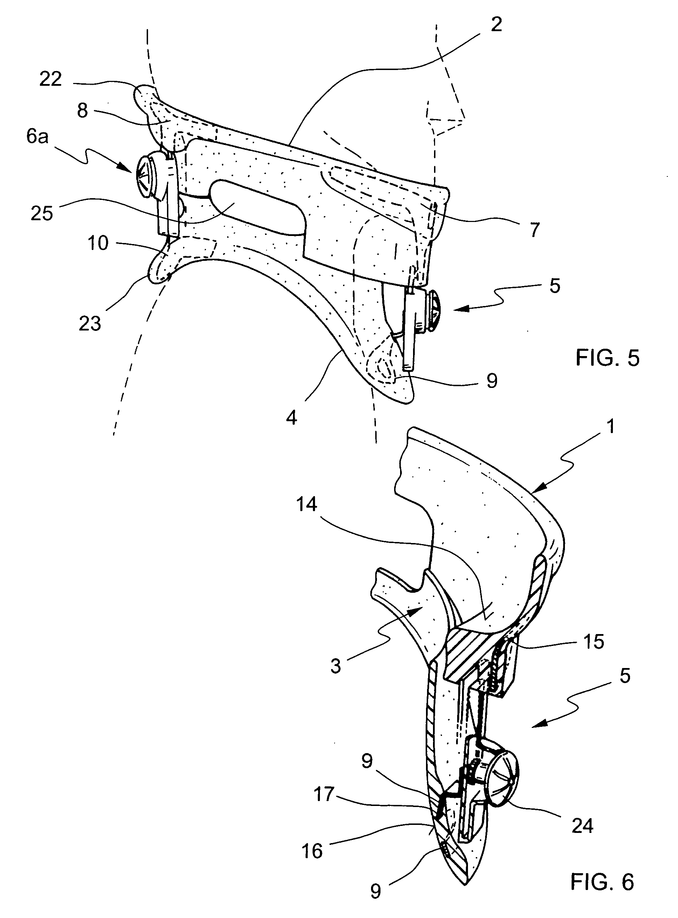

The cervical collar shown in Figures 1 to 6 is formed by two upper 1 and lower 3 flexible bodies each one having the general shape of a flexible strap obtained molded as a single part of plastic material. These flexible straps 1, 3 have a flexibility in their longitudinal direction which allows opening them by separating their ends, in which closing means 20, 21 of Velcro® type, are provided. The collar opens then by its rear portion, which is arranged in the occipital area.

The lower flexible body 3 is joined to in and has a vertical movement in the upper flexible body 1. The two flexible bodies 1 and 3 are attached by three extension mechanisms : an anterior extension mechanism 5 arranged in an anterior centered position with respect to the longitudinal medium plane of the collar and two rear extension mechanisms 6a, 6b arranged in rear positions decentered and symmetric with respect to said longitudinal medium plane. Each of these extension mechanisms 5, 6a, 6b is provided with an actuator 11, 12 that adjusts and maintains the vertical distance between an upper rigid part 7, 8 and a lower rigid part 9, 10, the ends of these rigid parts being fixedly attached to the upper 1 and lower 3 flexible bodies by overmolding.

In this example of specific embodiment, the rigid parts 7, 8, 9 and 10 are made of metal. Nevertheless, it is possible to manufacture these parts in a plastic material of high rigidity and mechanical strength, and to manufacture likewise in plastic material all the elements of the extension mechanisms 5, 6a, 6b, thereby making the collar according to the invention compatible with the X-ray devices, and radiographs with the collar in its use position can be made.

As it can be seen with more detail in Fig. 1, each actuator 11, 12 comprises a manually driven rotating slide 24 provided with a pinion geared with a serration of said upper 7, 8 and lower 9, 10 rigid parts, so that the rotating movement of the slide 24 causes a relative vertical movement between said rigid parts. The slide 24 is provided with a locking mechanism (not shown in the figure) that provides a rest position wherein the slide 24 is blocked and is capable of bearing a force of at least 20 N tending to shorten the distance between the upper 7, 8 and lower 9, 10 rigid parts. This locking mechanism is carried out for example with a rotating slide 24 which is slightly movable in the axial direction and it is provided with a spring which keeps the locking mechanism in an outlet axial position wherein a flange prevents its rotating movement. In order to unlock the slide 24, the slide is slightly pushed against the action of the spring, releasing it thereby from the flange. Optionally, each of the extension mechanisms 5, 6a, 6b is provided with a dynamometer 27 which indicates the force applied between the upper rigid part 7, 8 and the lower rigid part 9, 10, that is the extending force made by the extension mechanism. In order not to complicate more the drawings, this dynamometer 27, which can be either digital or mechanical, is schematically shown, in dash line, only in the anterior extension mechanism of Fig. 1.

The upper rigid part 7 of the anterior extension mechanism 5 has the shape of an arm that bifurcates into two symmetric branches 13 with the shape of a fork that rest in the upper flexible body 1, extending backwards within a closed edge 22 defined in the upper edge 2 of the upper rigid part 7. On the other hand, the upper flexible body 1 defines, in an anterior centered position with respect to the longitudinal medium plane of the collar, a concave base surface 14 arranged underneath said upper edge 2. The upper rigid part 7 rests, at the area 15 of bifurcation of the symmetric branches 13, underneath said concave base surface 14. Thanks to this configuration, in the use position of the collar, the anterior part of the upper flexible body 1 rests at its upper edge 2, at the level of said symmetric branches 13, in the lateral area of the jaws of the patient, and at the same time said anterior part also rests in the chin of the patient at the concave base surface 14.

In turn, the lower rigid part 9 of the anterior extension mechanism 5 has an arm shape that in its lower end comprises a portion 17 with a loop shape which rests on the lower flexible body 3 at the level of a base surface 16 defined therein. This base surface 16 is in an anterior centered position with respect to the longitudinal medium plane of the collar and it is arranged separated from the lower edge 4 of said lower flexible body 3. Thanks to this configuration, in the use position of the collar, the anterior portion of the lower flexible body 3 rests in the pectoral area of the patient both at its lower edge 4 and at said base surface 16.

The anterior extension mechanism 5 so constituted applies in a particularly efficient fashion an extension between the chin and the anterior pectoral area of a user wearing the collar.

In turn, the upper rigid parts 8 of the rear extension mechanisms 6a, 6b have the shape of an arm that bifurcates into two branches 18 which rest in the upper flexible body 1 extending in opposite directions within a closed rim 22 defined along the upper edge 2.

Likewise, the lower rigid parts 10 of the rear extension mechanism 6a, 6b have the shape of an arm that bifurcates into two branches 19 which rest in the lower flexible body 3 extending in opposite directions within a closed rim 23 defined along the lower edge 4.

The rear extension mechanisms 6a, 6b so constituted apply in a particularly efficient fashion an extension between the occipital area of the head and the rear scapular area of the trunk of a user, thereby obtaining a traction of the cervical column, and at the same time said mechanisms allow to apply in an accurate and controlled fashion a lateral unbalance of said traction.

By properly adjusting the extension mechanisms 5, 6a and 6b by means of the slides 24, a physiotherapist or a physician will be able to adjust in a controlled and accurate fashion any combination of pure traction of the cervical column, flexion or extension of the chin and right or left lateral inclination.

## Claims

1. Portable cervical collar suitable for exerting a therapeutical cervical traction, said cervical collar comprising an upper flexible body (1) having an upper edge (2) foreseen to rest in the head of a user wearing the collar, a lower flexible body (3) having a lower edge (4) foreseen to rest in the trunk of said user, said upper (1) and lower (3) flexible bodies being joined together allowing a relative vertical movement, and extension mechanisms (5, 6a, 6b) which are a part with the cervical collar and each one comprising an upper rigid part (7, 8) which rests in said upper flexible body (1), a lower rigid part (9, 10) that rests in said lower flexible body (3) and a manually adjustable actuator (11, 12) for adjusting and maintaining the relative vertical position between said upper (7, 8) and lower (9, 10) rigid parts, **characterized in that** it comprises three of said extension mechanisms (5, 6a, 6b): one anterior extension mechanism (5) arranged in an anterior centered position with respect to the longitudinal medium plane of said collar and two rear extension mechanisms (6a, 6b) arranged in rear positions decentered and symmetric with respect to said longitudinal medium plane of the collar, so that said anterior extension mechanism (5) is suitable for applying an extension between the chin and the anterior pectoral area of a user wearing the collar and said rear extension mechanisms (6a, 6b) are suitable for applying an extension between the occipital area of the head and the rear scapular area of the trunk of said user.

2. Cervical collar according to claim 1, **characterized in that** said upper rigid part (7) of the anterior extension mechanism (5) has the shape of an arm which bifurcates into two symmetric branches (13) which rest in said upper flexible body (1) extending backwards along its upper edge (2).

3. Collar according to claims 1 or 2, **characterized in that** said upper flexible body (1) defines, in an anterior centered position with respect to said longitudinal medium plane of the collar, a concave base surface (14) suitable for supporting the chin of a user wearing the collar, said concave base surface (14) being arranged underneath the upper edge (2) of said upper flexible body (1) and said upper rigid part (7) of the anterior extension mechanism (5) having a portion (15) at which said part rests in said upper flexible body (1) under said concave base surface (14).

4. Collar according to claims 2 and 3, **characterized in that** said symmetric branches (13) of the upper rigid part (7) of the anterior extension mechanism (5) have the shape of a fork defining, in the bifurcation of said symmetric branches (13), said area (15) at which the upper rigid part (7) of the anterior extension mechanism (5) rests under said concave base surface (14).

5. Collar according to any of claims 1 to 4, **characterized in that** said lower flexible body (3) defines, separated from its lower edge (4) and in an anterior centered position with respect to said longitudinal medium plane of the collar, a base surface (16) suitable for resting in the pectoral area of a user wearing the collar, and said lower rigid part (9) of the anterior extension mechanism has a portion (17) at which said rigid part rests in said lower flexible body (3) at the level of said base surface (16).

6. Collar according to claim 5, **characterized in that** said lower rigid part (9) of the anterior extension mechanism (5) has the shape of an arm which in its lower end has the shape of a loop defining said portion (17) at which the lower rigid part (9) rests in the lower flexible body (3) at the level of the base surface (16).

7. Collar according to any of claims 1 to 6, **characterized in that** said upper rigid parts (8) of the rear extension mechanisms (6a, 6b) have the shape of an arm which bifurcates into two branches (18) which rest in said upper flexible body (1) extending in opposite directions along their upper edge (2), and said lower rigid parts (10) of the rear extension mechanisms (6a, 6b) have the shape of an arm which bifurcates into two branches (19) that rest in said lower flexible body (3) extending in opposite directions along their lower edge (4).

8. Collar according to any of claims 1 to 7, **characterized in that** each of said upper (1) and lower (3) flexible bodies is molded as a single part overmolding the end portions of said upper (7, 8) and lower (9, 10) rigid parts of the extension mechanisms (5, 6a, 6b).

9. Collar according to claims 2, 7 and 8, **characterized in that** the upper edge (2) of said upper flexible body (1) has the shape of a closed rim (22) housing said branches (13, 18) of the upper rigid parts of the anterior (5) and rear (6a, 6b) extension mechanisms, and the lower edge (4) of said lower flexible body (3) has the shape of a closed rim (23) housing said branches (19) of the lower rigid parts of the rear extension mechanisms (6a, 6b).

10. Collar according to any of claims 1 to 9, **characterized in that** each of said upper (1) and lower (3) flexible bodies has the general shape of a flexible strap the ends of which are gathered in the rear portion of the collar and said strap having closing means (20, 21) of said ends on themselves.

11. Collar according to any of claims 1 to 10, **characterized in that** each of said actuators (11, 12) comprises a manually movable slide (24) the movement of which is mechanically linked to the relative movement of said upper (7, 8) and lower (9, 10) rigid parts, said slide (24) being suitable for firmly keeping a relative position between said upper (7, 8) and lower (9, 10) rigid parts against a force higher than or equal to 20 N and said slide (24) having a locking position of said relative movement.

12. Collar according to any of claims 1 to 11, **characterized in that** said upper (1) and lower (3) flexible bodies define two wide lateral windows (25) allowing access to the lateral areas of the neck of a user wearing the collar and a wide rear window (26) allowing access to the cervical area of the neck of said user.

13. Collar according to any of claims 1 to 12, **characterized in that** each of said extension mechanims (5, 6a, 6b) is provided with an indicator of the force applied between the upper rigid part (7, 8) and the lower rigid part (9, 10).
